# EUROPÄISCHE PATENTSCHRIFT

(11) **EP 1 498 492 B1**
(45) Veröffentlichungstag und Bekanntmachung des Hinweises auf die Patenterteilung: **02.11.2011**
(21) Anmeldenummer: 03016057.6
(22) Anmeldetag: 15.07.2003
(51) Int. Cl.: C12Q 1/68

(54) **PROBENZUBEREITUNGSEINHEIT**
APPARATUS FOR THE PREPARATION OF SAMPLES
APPAREIL POUR LA PREPARATION DES ECHANTILLONS

(43) Veröffentlichungstag der Anmeldung: 19.01.2005
(73) Patentinhaber: Bestmann, Lukas, 8406 Winterthur (CH); Dual, Jürg, 8126 Zumikon (CH)
(72) Erfinder: Bestmann, Lukas, 8406 Winterthur (CH)
(74) Vertreter: Hepp Wenger Ryffel AG

(56) Entgegenhaltungen:
- WO-A-01/92569
- US-A- 5 861 251
- US-A- 6 136 555
- US-B1- 6 300 073

## Beschreibung

Die vorliegende Erfindung betrifft ein Lyophilisat und eine Einheit enthaltend dieses Lyophilisat zur Zubereitung von Reaktionsmischungen, insbesondere von Polynukleotiden für eine chemische Reaktion wie die PCR-Reaktion (Polymerase Chain reaction), sowie ein Verfahren zur Zubereitung derartiger Reaktionsmischungen unter Verwendung einer solchen Einheit.

Die Isolierung und Analyse von Polynukleotiden wie DNA und RNA spielt eine zentrale Rolle in der mikrobiologischen und genetischen Diagnostik. Für die Identifizierung von Genen in Zellen, von Bakterien oder Viren in einer Probe oder die Erkennung von Mutationen ist eine Isolierung und/oder Reinigung der Polynukleotide erforderlich, um zuverlässige Ergebnisse zu erhalten.

Herkömmliche Verfahren zur Zubereitung von polynukleotidhaltigen Proben umfassen den Schritt der Lyse von zu untersuchenden Zellen, und die nachfolgende Trennung störender Zellbestandteile von den Polynukleotiden. Die bekannten Trennverfahren lassen sich in zwei allgemeine Kategorien einordnen. Bei der einen Kategorie von Trennverfahren wird eine polynukleotidhaltige Lösung dadurch gereinigt, dass störende Bestandteile durch beispielsweise Ausfällung und/oder Zentrifugation von den Polynukleotiden abgetrennt werden (Flüssigphasenreinigung). Bei der anderen Kategorie von Trennverfahren werden die Polynukleotide an einen festen Träger gebunden und die störenden Bestandteile der Probe durch Auswaschen mit geeigneten Lösungsmitteln entfernt (Festphasenreinigung).

Die gängigsten Verfahren der Flüssigphasenreinigung von Polynukleotidproben beinhalten die folgenden 3 Schritte:
1. Lyse von Zellen zur Freisetzung der Polynukleotide
2. Abtrennung störender Bestandteile der Probe aus dem flüssigen Gemisch
3. Aufkonzentrierung des verbleibenden Gemisches (z.B. durch alkoholische Fällung) und erneutes in Lösung bringen der Polynukleotide

Der zweite Schritt kann beispielsweise durch Zentrifugation, Extraktion mit organischen Lösungsmitteln, Ausfällung oder Chromatographie erfolgen.

Die gängigsten Verfahren der Festphasenreinigung von Polynukleotidproben beinhalten die folgenden 3 Schritte:
1. Lyse von Zellen zur Freisetzung der Polynukleotide
2. Binden der Polynukleotide an einen festen Träger
3. Entfernen von störenden Bestandteilen der Probe durch Waschen mit geeigneten Lösungsmitteln
4. Eluieren der Polynukleotide vom festen Träger

Als fester Träger werden beispielsweise Membranfilter, Metalloxide, Latex-Partikel oder magnetische Kugeln vorgeschlagen.

Die vorstehend beschriebenen Verfahren weisen einige Nachteile auf. In der Regel sind mehrere Verfahrensschritte in unterschiedlichen Vorrichtungen durchzuführen. Zudem verlangen diese Verfahren den Einsatz von verschiedenen, teilweise nicht unbedenklichen Lösungsmitteln wie Phenolen oder halogenierten Lösungsmitteln. Die herkömmlichen Verfahren sind zeitraubend, kompliziert und verlangen den Einsatz von Stoffen zur Reinigung, die nachträglich möglichst quantitativ wieder entfernt werden müssen, da sie nachträgliche chemische Reaktionen wie die Polymerase-Kettenreaktion (PCR) beziehungsweise die nachfolgende Analyse der Reaktionsmischungen behindern.

Es sind Versuche beschrieben, das Verfahren zur Zubereitung polynukleotidhaltiger Reaktionsgemische insbesondere für die PCR zu vereinfachen. In der WO 99/39009 ist ein Verfahren beschrieben, bei dem in einer Einheit die aufzureinigende Probe zunächst mit einem festen Träger in Kontakt gebracht wird. An diesem festen Träger, beispielsweise eine Membran oder ein Filter, ist ein Lysemittel gebunden. Die Lyse erfolgt somit an diesem festen Träger. Anschliessend wird die in dem Gemisch enthaltene DNA aufgereinigt, indem das Gemisch mit einem DNA-Aufreinigungsmittel wie einem organischen Lösungsmittel behandelt wird. Für den Fall, dass die DNA hierbei an einem festen Träger gebunden ist, wird das Polynukleotid anschliessend von dem Träger eluiert. Bei diesem Verfahren ist ein Reinigen des Reaktionsgemisches mit organischen Reagenzien erforderlich. Zudem wird als Eluiermittel eine Zusammensetzung verwendet, die einen Chelatbildner enthält. Derartige Substanzen stören die PCR und eine nachfolgende Detektion in einem erheblichen Masse beziehungsweise verhindern derartige Folgeschritte vollständig.

In der WO 95/02049 ist ein Verfahren beschrieben, bei dem in einer Einheit eine zu analysierende Probe zunächst filtriert wird, um flüssige Bestandteile abzutrennen. Anschliessend werden die auf dem Filter gesammelten Zellen resuspendiert und lysiert. Die lysierte Mischung wird anschliessend in eine zweite Kartuschenkammer filtriert. Das gefilterte Lysat wird in dieser Kammer unter Bedingungen auf eine feste Matrix gebracht, unter denen es zu einer Bindung der DNA an die feste Matrix kommt. Bei der festen Matrix handelt es sich gemäss den Beispielen der WO 95/02049 um ein Ionenaustauscherharz. Dieses Harz wird in der zweiten Kammer der Kartusche in Form einer Suspension bereitgestellt. Die Suspension wird mit Waschlösungen behandelt, um störende Bestandteile abzutrennen und durch einen Filter zu entfernen. Anschliessend wird die DNA mit einem Eluiermittel von der Matrix entfernt und ebenfalls durch den Filter aus der Kartusche herausgespült. Bei diesem Verfahren müssen somit ebenfalls mehrere verschiedene Lösungen zum Suspendieren und Waschen des Reaktionsgemisches verwendet werden.

Allen vorstehend beschriebenen Verfahren ist zu eigen, dass sie nur ein gereinigtes Polynukleotid bereitstellen. Es ist kein Verfahren beziehungsweise keine Kartusche beschrieben, durch welche(s) eine vollständige Reaktionsmischung für die PCR in einem einzigen Arbeitsvorgang bereitgestellt werden kann.

Es war die Aufgabe der vorliegenden Erfindung, eine Einheit und ein Verfahren bereitzustellen, um eine polynukleotidhaltige Reaktionsmischung für die PCR auf einfache Weise aus einer biologischen Probe bereitzustellen.

Die vorstehende Aufgabe wird gemäss der vorliegenden Erfindung durch eine, Zusammensetzung zur Durchführung einer chemischen Reaktion, einer Einheit beziehungsweise ein Verfahren sowie eine Vorrichtung wie in den unabhängigen Ansprüchen definiert gelöst.

Die vorliegende Erfindung betrifft eine Einheit, vorzugsweise eine Kartusche zur Zubereitung von Reaktionsgemischen für chemische Reaktionen, insbesondere für die Polymerase-Kettenreaktion, umfassend einen Einlass und einen Auslass sowie mindestens einen Träger, vorzugsweise eine Membran, dadurch gekennzeichnet, dass an mindestens einen Träger eine nachstehend genauer definierte Zusammensetzung in fester Form gebunden ist, welche zur Durchführung der chemischen Reaktion erforderliche Substanzen enthält.

Die erfindungsgemässe Einheit hat den Vorteil, dass in einem einfachen und schnellen Arbeitsvorgang ein Reaktionsgemisch für eine chemische Reaktion bereitgestellt werden kann. Die zu analysierende Substanz wird in den Einlass der Einheit gegeben. Beim Durchtritt durch den in der Einheit befindlichen Träger, bei welchem es sich vorzugsweise um eine Membran handelt, wird die Substanz mit einer nachstehend genauer definierten Zusammensetzung vermischt, welche vorzugsweise an der Unterseite einer Membran bereitgestellt ist. Aus dem Auslass der Einheit tritt somit das fertige Reaktionsgemisch aus. Bei der Zusammensetzung handelt es sich erfindungsgemäss bevorzugt um ein Lyophilisat.

Die vorliegende Erfindung ist nicht auf eine bestimmte Art von Probenzubereitungseinheit eingeschränkt. Grundsätzlich sind von der vorliegenden Erfindung sämtliche Einheiten umfasst, in welchen eine Probe in Kontakt mit einem Träger kommen kann, an den eine Zusammensetzung gebunden ist. Erfindungsgemäss bevorzugt sind Einheiten, die in vertikaler Richtung von der Probe durchströmt werden können. Erfindungsgemäss besonders bevorzugt ist die Einheit eine Kartusche.

Gemäss der vorliegenden Erfindung ist unter einem Träger jedes feste Material zu verstehen, an welches die Zusammensetzung gebunden werden kann. Beispielsweise kann der Träger eine Membran, eine oder mehrere Kugeln oder eine lockere Schicht sein. Erfindungsgemäss bevorzugt ist der Träger eine Membran. Unter einer Membran soll gemäss der vorliegenden Erfindung eine für Flüssigkeiten und Feststoffe wenigstens teildurchlässige Wand oder Dünnschicht zu verstehen sein. Besonders bevorzugt ist die Membran ein Filter.

Der Durchtritt der Probe durch die Einheit kann durch Anlegen eines Überdrucks beziehungsweise Unterdrucks an die Einheit beschleunigt werden. Dies kann durch Bereitstellung entsprechender Öffnungen in der Einheit erreicht werden. Soll beispielsweise mit Hilfe von Überdruck die Probe schneller durch die Membran gedrückt werden, ist eine Öffnung zum Anlegen eines Überdrucks im Bereich der Einheit vom Einlass bis zur Membran bereitzustellen. Bei der Öffnung kann es sich auch um den Einlass der Einheit selbst handeln. An diese Öffnung kann ein Gerät zur Erzeugung eines Überdrucks an die Einheit angeschlossen werden. Beispielsweise kann es sich bei diesem Gerät um eine herkömmliche Pumpe handeln. Es kann aber auch eine Spritze angebracht werden, mit deren Hilfe Luft in die Einheit gedrückt werden kann. Soll mit Hilfe von Unterdruck die Probe schneller durch die Membran gezogen werden, ist eine Öffnung zum Anlegen eines Unterdrucks im Bereich der Einheit von der Membran bis zum Auslass der Einheit bereitzustellen. An diese Öffnung kann ein Gerät zur Erzeugung eines Unterdrucks an die Einheit angeschlossen werden. Beispielsweise kann es sich bei diesem Gerät um eine herkömmliche Vakuumpumpe handeln. Es kann aber auch eine Spritze angebracht werden, mit deren Hilfe Luft aus der Einheit gezogen werden kann. Die anzulegende Druckerhöhung beziehungsweise Druckerniedrigung kann vom Fachmann leicht bestimmt werden.

Gemäss der vorliegenden Erfindung ist unter einer Probe die zu verarbeitende Zusammensetzung zu verstehen. Diese enthält im Fall der Zubereitung von Reaktionsgemischen für eine PCR Polynukleotide. Die Probe kann Körperflüssigkeit, beispielsweise Blut oder Speichel, Pflanzensaft oder eine ähnliche biologische Quelle oder eine synthetische Zusammensetzung sein.

Ein Reaktionsgemisch für die Polymerase-Kettenreaktion (PCR) enthält beispielsweise neben dem zu amplifizierenden Polynukleotid wie DNA das Enzym Polymerase zur Katalysierung der Amplifizierungsreaktion, Desoxyribonukleotidtriphosphate (dNTPs) als Bausteine der zu synthetisierenden Polynukleotide, Oligonukleotid-Primer zur Auslösung der Reaktion sowie gegebenenfalls weitere Stoffe wie Puffer. Die herkömmliche Zubereitung eines Reaktionsgemisches für die PCR nimmt aufgrund des sorgfältigen Abmessens der Mengen der zahlreichen Bestandteile und des Vermischens nicht unerheblich Zeit in Anspruch. Dieser Zeit- und Arbeitsaufwand entfällt bei der erfindungsgemässen Einheit und dem erfindungsgemässen Verfahren.

Die erfindungsgemässe Einheit kann über einen langen Zeitraum bei Raumtemperatur (25°C) gelagert werden. Sie ist für den einmaligen Gebrauch vorgesehen.

Bei bestimmten biologischen Proben wie Blutproben muss vor dem Vermischen mit den übrigen Bestandteilen des Reaktionsgemisches für die PCR das Polynukleotid durch Lyse aus den Zellen freigesetzt und von den anderen störenden Bestandteilen der biologischen Probe gereinigt werden. Dies kann mit Hilfe einer weiteren Ausführungsform der erfindungsgemässen Einheit ebenfalls in einem Arbeitsschritt auf einfache Weise durchgeführt werden. Gemäss der vorliegenden Erfindung werden hierfür in der Einheit zwischen dem Einlass und dem Träger, an welchen die Zusammensetzung gebunden ist, zusätzlich 1 oder mehrere Membranen oder Träger, vorzugsweise 4 Membranen bereitgestellt. Diese Träger, vorzugsweise Membranen, sind vorzugsweise so angeordnet, dass sich ihre Porengrösse mit zunehmender Entfernung vom Einlass der Einheit verkleinert. Auf diese Weise kann eine schrittweise Abtrennung immer kleinerer Bestandteile von der Probe erfolgen, ohne dass die Gefahr einer Verstopfung einer Membran besteht. Eine weitergehende Reinigung kann erreicht werden, wenn in einem Zwischenraum zwischen zwei Membranen eine Festkörper absorbierende Substanz wie beispielsweise Aerosil bereitgestellt wird.

Gemäss einer bevorzugten Ausführungsform der vorliegenden Erfindung ist einer der zusätzlichen Träger so ausgebildet, dass sich Polynukleotide an ihm binden. Dies kann beispielsweise durch Bereitstellung entsprechender funktioneller Gruppen an einer Membran erfolgen. Erfindungsgemäss bevorzugt werden an einer Membran funktionelle Gruppen bereitgestellt, die eine positive Ladung tragen und/oder ausbilden können. Als Beispiel seien Aminogruppen wie die Diethylaminoethylgruppe genannt. Andere Probenbestandteile können dann von den Polynukleotiden abgetrennt werden. Die Polynukleotide werden anschliessend mit Hilfe eines Eluiermittels von dem Träger, vorzugsweise der Membran eluiert. Grundsätzlich kann das Eluiermittel durch den Einlass oder eine beliebige andere in der Einheit vorhandene Öffnung oberhalb des vorstehenden Trägers in die Einheit eingeführt werden. Erfindungsgemäss bevorzugt umfasst die Einheit jedoch eine Einheit zur Zuführung einer Flüssigkeit wie dem Eluiermittel. Diese Einheit weist einen Vorratsbehälter auf, in welchem das Eluiermittel gelagert ist. Der Vorratsbehälter ist vom Innenraum der Einheit durch eine Membran getrennt. Bei intakter Membran ist ein Eintritt des Eluiermittels in die Einheit nicht möglich. Durch Anlegen eines Unterdrucks kann diese Membran zerstört werden, wodurch das Eluiermittel in die Einheit gelangt.

Bei den in der Einheit enthaltenen Membranen handelt es sich erfindungsgemäss bevorzugt um Filter, d.h. um zur Filtration geeigneten Werkstoffen. Jeder herkömmlich verwendete Filter kann grundsätzlich für die erfindungsgemässe Einheit verwendet werden. Die Filter können beispielsweise aus Cellulose, Cellulosederivaten wie Celluloseacetat, beispielsweise Zeolith, Kunststoffen wie Polyamiden, beispielsweise Nylon, Polysulfonen oder halogenierten Polymeren, beispielsweise Polytetrafluorethylen oder Polyvinylchlorid, gefertigt sein. Vorzugsweise weisen die in der erfindungsgemässen Einheit verwendeten Filter Porengrössen in einem Bereich von einschliesslich 5 µm bis einschliesslich 0,2 µm, vorzugsweise 0,3 bis 0,9 µm auf. Als Limitierung ist bei einer bevorzugten Ausführungsform der vorliegenden Erfindung zu berücksichtigen, dass sich die Porengrösse der verwendeten Filter mit grösserem Abstand vom Einlass der Einheit graduell verringern soll.

Der Träger, der zur Bindung von Polynukleotiden vorgesehen ist, ist vorzugsweise ein Filter und muss so ausgebildet sein, dass er über Wechselwirkungen mit Polynukleotiden diese Substanzen binden kann. Es können hierfür beispielsweise ionische Wechselwirkungen oder van der Waals-Kräfte ausgenützt werden. Erfindungsgemäss bevorzugt werden ionische Wechselwirkungen herangezogen. Polynukleotide sind bei physiologischem pH-Wert negativ geladene Moleküle. Durch Bereitstellung von funktionellen Gruppen auf einer Membran, welche eine positive Ladung besitzen und/oder ausbilden können, kann die gewünschte Bindung der Polynukleotide an die Membran erreicht werden. Es können hierfür herkömmliche KationenAustauscherharze als Material für den Filter oder zur Imprägnierung eines Filters aus einem der vorstehend aufgeführten Materialien verwendet werden. Erfindungsgemäss bevorzugt weist diese Membran Aminogruppen wie Diethylaminoethyl-(DEAE)-Gruppen auf.

Gemäss einer bevorzugten Ausführungsform der vorliegenden Erfindung können eine oder mehrere der vorstehend beschriebenen Membranen oder Träger mit einer Substanz imprägniert sein, welche die Oberflächenspannung von Flüssigkeiten erhöhen kann. Dies führt einerseits zu einem zusätzlichen Filtereffekt und andererseits zu einem besseren Fliessverhalten des Reaktionsgemisches. Es können alle Substanzen eingesetzt werden, die den entsprechenden Effekt auf die Oberflächenspannung ausüben und zur Imprägnierung einer Membran geeignet sind. Erfindungsgemäss bevorzugt werden hierfür polymere Siliciumverbindungen verwendet. Erfindungsgemäss besonders bevorzugt werden Polydimethylsiloxane wie Dimeticon® eingesetzt.

Für den Fall, dass Einheiten zur Aufreinigung der Probe vorhanden sind, umfasst die erfindungsgemässe Einheit weiterhin einen Abfallbehälter zur Aufnahme der Probenbestandteile,
die nicht in das Reaktionsgemisch gelangen sollen. Mit Hilfe einer Reguliereinheit wie einem Dreiwegehahn kann wahlweise Substanz über entsprechend bereitgestellte Zuführungsleitungen in den Abfallbehälter oder in eine Auffangvorrichtung für das Reaktionsgemisch geleitet werden.

Wenn eine Lyse erforderlich ist, kann diese wahlweise durch Zugabe eines Lysemittels durch den Einlass oder eine beliebige andere vorhandene Öffnung der Einheit zur Probe oder durch Kontaktieren der Probe mit einer mit Lysemittel imprägnierten Membran erfolgen.

Bei allen Ausführungsformen der vorliegenden Erfindung kann oberhalb des Einlasses eine Kapillare bereitgestellt sein. Dadurch kann ein definiertes Volumen einer flüssigen Probe in die Einheit eingeführt werden.

Die erfindungsgemässe Einheit kann aus jedem herkömmlichen für derartige Einheiten verwendeten Material gefertigt sein. Erfindungsgemäss bevorzugt ist die Verwendung von Glas als Material für die Wände der Einheit.

Die erfindungsgemässe Einheit hat typischerweise eine Grösse von etwa 3 cm.

Gemäss der vorliegenden Erfindung wird weiterhin ein Verfahren zur Zubereitung von Reaktionsgemischen für chemische Reaktionen, insbesondere für die Polymerase-Kettenreaktion, bereitgestellt, umfassend die Schritte:
a) Einführen einer Probe in die Einheit gemäss einem der Ansprüche 1 bis 14;
b) Durchtritt der Probe durch einen Träger, vorzugsweise eine Membran, an welche eine nachstehend genauer definierte Zusammensetzung gebunden ist, die zur Durchführung der chemischen Reaktion erforderliche Substanzen enthält, so dass das fertige Reaktionsgemisch aus dem Auslass der Einheit austritt.

Auf diese Weise kann ein Reaktionsgemisch innerhalb eines kurzen Zeitraums von typischerweise 3 bis 5 Minuten bereitgestellt werden. Die insbesondere bei der PCR zeitaufwendige und arbeitsintensive Herstellung des Reaktionsgemisches mit zahlreichen Mess-, Pipettier-, Verdünnungs- und Mischschritten entfällt beim erfindungsgemässen Verfahren. Das Reaktionsgemisch mit Ausnahme der zu analysierenden Polynukleotide wird in Form einer Zusammensetzung in fester Form auf einem Träger, vorzugsweise einer Membran bereitgestellt. Vorzugsweise handelt es sich bei der Zusammensetzung um ein Lyophilisat. Diese enthält im Fall eines Reaktionsgemisches für die PCR das Enzym DNA-Polymerase zur Katalysierung der Amplifizierungsreaktion, Desoxyribonukleotidtriphosphate (dNTPs) als Bausteine der zu synthetisierenden Polynukleotide, Oligonukleotid-Primer zur Auslösung der Reaktion sowie gegebenenfalls weitere Stoffe wie Puffer. Erfindungsgemäss können hierfür grundsätzlich alle Reaktionsmischungen verwendet werden, die sich lyophilisieren lassen. Als Beispiel seien die in der US-5,861,251 und US-6,153,412 beschriebenen Zusammensetzungen genannt.

Eine erfindungsgemäss bevorzugt einsetzbare Zusammensetzung für eine PCR ist die nachfolgend beschriebene Mischung. Diese enthält eine Polymerase, beispielsweise DNA-Polymerase, vorzugsweise Taq-Polymerase. Taq-Polymerase ist eine aus dem Organismus Thermos aquaticus stammende DNA-Polymerase und ist die am schnellsten amplifizierende Polymerase.

Die Art der verwendeten Polymerase ist abhängig von der zu amplifizierenden Substanz. Dem Fachmann sind die entsprechenden Polymerasen für die jeweiligen Targets bekannt.

Die Polymerase wird in einer Stammlösung vorgegeben, welche zusätzlich eine Pufferlösung, vorzugsweise Tris-Puffer mit einem pH-Wert von 8,3 und Rinderserumalbumin (BSA) enthält.

Die Stammlösung wird anschliessend mit einer Pufferlösung, mindestens einem Alkaliund/oder Erdalkalimetallhalogenid, den entsprechenden Desoxyribonukleotidtriphosphaten (dNTPs), mindestens einem Primer, einem Stabilisator sowie Stoffen zur Detektion des Reaktionsprodukts und gegebenenfalls weiteren Zusatzstoffen versetzt. Die Lösung wird anschliessend gegebenenfalls mit Wasser verdünnt.

Die Pufferlösung ist vorzugsweise Tris-Puffer mit einem pH-Wert von 8,3. Daneben enthält die Pufferlösung vorzugsweise Rinderserumalbumin (BSA) und MgCl₂. Der Pufferlösung können zudem zusätzliche Substanzen wie beispielsweise Ficoll zugegeben werden.

Die Zusammensetzung enthält als mindestens ein Metallhalogenid MgCl₂. Daneben kann die Zusammensetzung weitere Alkali- und/oder Erdalkalimetallhalogenide, vorzugsweise Chloride, besonders bevorzugt KCl enthalten.

Die Zusammensetzung für die PCR muss mindestens einen Primer enthalten. Ein Primer ist eine Oligonukleotidsequenz, welche an das zu amplifizierende Polynukleotid anhaftet und für die Arbeit der Polymerase notwendig ist. Im Fall der Amplifizierung von doppelsträngiger DNA sind zwei Primer erforderlich. Die Primer sind in Abhängigkeit von dem zu amplifizierenden Polynukleotid auszuwählen. Entsprechende Primer sind dem Fachmann bekannt. Erfindungsgemäss bevorzugt sind Primer, die aus Abschnitten der 16SRNA bestehen.

Die Zusammensetzung enthält weiterhin einen Stabilisator. Der Stabilisator stabilisiert die Polymerase bei der Lyophilisation und ermöglicht eine Langzeitlagerung der Zusammensetzung ohne nennenswerten Aktivitätsverlust des Enzyms. Die Lyophilisation von Proteinen sowie hierfür verwendete Stabilisatoren sind bekannt. Der Stabilisator ist für die Aufrechterhaltung der Tertiärstruktur des Proteins während der Lyophilisation verantwortlich. Bekanntlich ist die Tertiärstruktur eines jeden Proteins unterschiedlich. Es kann somit nicht verlässlich vorhergesagt werden, welcher Stabilisator für welches Protein geeignet ist. Es wurde gefunden, dass zur Stabilisierung von Taq-Polymerase Disaccharide und insbesondere bevorzugt Trehalose geeignet ist. Erfindungsgemäss bevorzugt enthält daher eine Zusammensetzung mit Taq-Polymerase als Enzym ein Disaccharid und vorzugsweise Trehalose.

Die Zusammensetzung enthält weiterhin Stoffe zur Detektion des Reaktionsprodukts. Hierbei handelt es sich um Substanzen, die durch physikalische Methoden detektierbar sind und sich an das amplifizierte Polynukleotid binden oder haften. Durch Detektion der Substanz kann somit die Anwesenheit des Polynukleotids ermittelt werden. Herkömmlicherweise werden als derartige Substanzen Farbstoffe eingesetzt. Als Beispiele seien Farbstoffe für die Gelelektrophorese oder Farbstoffe für die Fluoreszenzmessung genannt. Erfindungsgemäss bevorzugt sind Stoffe, die sich fluorometrisch erfassen lassen. Derartige Farbstoffe sind dem Fachmann bekannt. Als Beispiel sind hier Fluorescein, Fluoresceinisothiocyanate, LC-Rot 640, LC-Rot 705, Rhodamin R6G, Oregon 488, Rhodolgrün oder FAM aufgeführt.

Erfindungsgemäss können jedoch auch Komponenten verwendet werden, die bei der FRET (fluorescence resonance energy transfer pair)-Technologie zum Einsatz kommen. Hierbei werden zwei unterschiedliche Oligonukleotide, die mit Fluorphoren verbunden sind und als Sensor und Anchor bezeichnet werden, der PCR-Reaktionsmischung zugegeben. Diese Oligonukleotide sind so auszuwählen, dass sie in räumlicher Nähe zueinander an das amplifizierte Polynukleotid anhaften können. Das Anchor-Oligonukleotid ist mit einem als Donor-Fluorophor bezeichneten Farbstoff verbunden. Wenn die amplifizierte Reaktionsmischung mit elektromagnetischer Strahlung entsprechender Wellenlänge bestrahlt wird, nimmt das Donor-Fluorphor die einfallende elektromagnetische Strahlung auf und transferiert diese auf das in räumlicher Nähe befindliche Akzeptor-Fluorophor. Das Akzeptor-Fluorophor ist vorzugsweise an das Sensor-Oligonukleotid gebunden und erzeugt aufgrund der vom Donor-Fluorophor transferierten elektromagnetischen Strahlung eine erfassbare Fluoreszenzemission. Ausführungsformen der FRET-Technologie sind beispielsweise in der US-6,174,670, der WO 92/14845 oder der EP-B-0 870 063 beschrieben.

Weiterhin muss gemäss der vorliegenden Erfindung der Zusammensetzung Carbopol 940 zugegeben werden, um oxidative Effekte zu verhindern.

Die Polymerase-Stammlösung, enthaltend die Polymerase, die Pufferlösung sowie Rinderserumalbumin, wird mit einer Pufferlösung, mindestens einem Alkali- und/oder Erdalkalimetallhalogenid, mindestens einem Primer, sowie einem Farbstoff (im Fall der Anwendung der FRET-Technologie mit einem Anchor- und einem Sensor-Oligonukleotid) und sanftem Rühren vermischt. Anschliessend werden die entsprechenden Desoxyribonukleotidtriphosphate (dNTPs), der Stabilisator sowie gegebenenfalls zusätzliche Komponenten zugegeben. Die so erhaltene Mischung wird anschliessend nach dem Fachmann bekannten Standard-Methoden lyophilisiert.

Das so erhaltene Lyophilisat zeichnet sich durch eine hohe Lagerstabilität aus. Es tritt nahezu kein Aktivitätsverlust der Polymerase auf.

Obwohl die Bereitstellung der Zusammensetzung in Form eines Lyophilisats die bevorzugte Ausführungsform der vorliegenden Erfindung darstellt, können jedoch auch andere dem Fachmann bekannte Möglichkeiten gewählt werden, um die vorstehende Zusammensetzung an den Träger zu binden.

Eine erfindungsgemäss besonders bevorzugte Reaktionsmischung umfasst unter anderem folgende Komponenten in folgenden Mengen:

| **Komponente** | **Menge** |
|---|---|
| Pufferlösung aus 50 mM Tris (pH=8.3), 0.25 mg/ml Rinder-serumalbumin, 0.5-1.0% Ficoll und 1 mM MgCl₂ | 2,0 µl |
| 50 mM KCl | 5,0 µl |
| 200 µM dNTP | 2,0 µl |
| Polymerase-Stammlösung (0,4 U/10 µl Polymerase, 250 µg/ml Rinderserumalbumin) | 2,0 µl |
| 0,5 µM Primer 1 | 1,0µl |
| 0,5 µM Primer 2 | 1,0 µl |
| 0,2 µM Sensor-Oligonukleotid | 0,2 µl |
| 0,4 µM Anchor-Oligonukleotid | 0,4 µl |
| 1,0 mM MgCl₂ | 0,8 µl |
| Trehalose | 8% (w/v) |

Während des Durchtritts der Probe durch vorzugsweise die Membran wird die besonders bevorzugt an der Unterseite der Membran befindliche Zusammensetzung von der Probenflüssigkeit mitgerissen beziehungsweise in dieser gelöst. Auf diese Weise tritt beim Auslass der Einheit ein fertiges Reaktionsgemisch aus, das neben dem zu amplifizierenden und zu untersuchenden Polynukleotid die Bestandteile der Zusammensetzung umfasst.

Der Durchtritt der Probe durch die Membran kann beschleunigt werden, indem man an der vom Auslass abgewandten Seite der Membran Überdruck anlegt oder an der dem Auslass zugewandten Seite der Membran Unterdruck anlegt. Dies kann durch die vorstehend beschriebenen Massnahmen durchgeführt werden.

Das vorstehend beschriebene einfache Verfahren kann nur mit Proben durchgeführt werden, bei denen weder eine Lyse von Zellen zur Freisetzung von Polynukleotiden noch eine Reinigung der Probe erforderlich ist. Bei der deutlichen Mehrzahl von biologischen Proben sind aber die vorstehend genannten Schritte erforderlich. Gemäss einer bevorzugten Ausführungsform der vorliegenden Erfindung umfasst daher das erfindungsgemässe Verfahren zwischen den Schritten a) und b) als zusätzliche Schritte
a1) eine Lyse von Zellen in der Probe
a2) die Trennung der Polynukleotide von anderen Probenbestandteilen.

Die Lyse von Zellen kann nach herkömmlichen Verfahren aus dem Stand der Technik durchgeführt werden. Gemäss einer Ausführungsform der vorliegenden Erfindung wird hierfür ein Lysemittel zur in der Einheit befindlichen Probe gegeben. Die erste Membran in der Einheit ausgehend vom Einlass weist eine derartige Porengrösse auf, dass Zellen nicht hindurchtreten können. Andererseits können die durch die Lyse der Zellen erhältlichen Teilchen durch diese Membran gelangen.

Eine weitere Ausführungsform der vorliegenden Erfindung besteht darin, dass der Schritt der Lyse dadurch erfolgt, dass die erste Membran in der Einheit ausgehend vom Einlass mit einem Lysemittel imprägniert ist. Wenn die zellhaltige Probe mit der Membran in Kontakt kommt, wird die Lyse der Zellen ausgelöst.

Herkömmliche und für die vorliegende Erfindung verwendbare Lysemittel umfassen beispielsweise ein Detergens. Es können anionische Detergentien wie N-Lauroylsarcosin oder Natriumdodecylsulfat verwendet werden. Weiterhin verwendbar sind enzymatische Lysemittel. Als erfindungsgemäss bevorzugtes Beispiel hierfür ist Proteinase K genannt. Nicht geeignet für das erfindungsgemässe Verfahren sind jedoch Lysemittel mit Bestandteilen, welche die nachfolgende chemische Reaktion und Detektion stören. Beispielsweise stören Chelatbildner eine nachfolgende PCR und deren Auswertung. Bei der Zubereitung von Reaktionsgemischen für die PCR sind daher Lysemittel mit chelatisierenden Bestandteilen zu vermeiden. Die Menge an Lysemittel ist abhängig von der im Probenvolumen enthaltenen Menge an Zellen. Erfindungsgemäss bevorzugt wird eine Menge von 1-10 µl Lysemittel bereitgestellt.

Bei dem erfindungsgemässen Verfahren durchläuft die Probe in der Einheit in Abhängigkeit von der Beschaffenheit der Probe und den Anforderungen an die Reinheit des bereitzustellenden Reaktionsgemisches mehrere zusätzliche Membranen oder Träger. Wie vorstehend beschrieben werden hierfür in der Einheit zwischen dem Einlass und dem Träger, an welche die Zusammensetzung gebunden ist, zusätzlich 1 oder mehrere Membranen oder Träger, vorzugsweise 4 Membranen bereitgestellt. Diese Membranen oder Träger sind vorzugsweise so angeordnet, dass sich ihre Porengrösse mit zunehmender Entfernung vom Einlass der Einheit verkleinert. Auf diese Weise kann eine schrittweise Abtrennung immer kleinerer Bestandteile von der Probe erfolgen, ohne dass die Gefahr einer Verstopfung einer Membran besteht. Die Porengrösse und Beschaffenheit der Membranen ist vorstehend beschrieben.

Aufgrund des Durchtritts durch die verschiedenen Membranen oder Träger wird ein zunehmender Reinheitsgrad der in der Probe befindlichen Polynukleotide erreicht. Durch die erfindungsgemässe Anordnung von Membranen oder Trägern bedarf es beim erfindungsgemässen Verfahren im Gegensatz zu den herkömmlichen Verfahren aus dem Stand der Technik keiner Waschschritte. Die Zugabe von Waschlösungen beinhaltet neben dem zusätzlichen Arbeitsaufwand grundsätzlich die Gefahr einer Kontamination des resultierenden Reaktionsgemisches. Derartige Schritte werden daher erfindungsgemäss vermieden.

Eine weitergehende Reinigung kann erreicht werden, wenn in einem Zwischenraum zwischen zwei Membranen eine Festkörper absorbierende Substanz wie beispielsweise Aerosil bereitgestellt wird. Auf diese Weise können Bestandteile wie das Hämoglobin aus einer Blutprobe entfernt werden, ohne dass es zu einer Verstopfung eines Filters kommt. Erfindungsgemäss bevorzugt ist diese Festkörper absorbierende Substanz zwischen den ersten beiden Membranen ausgehend von Einlass der Einheit angeordnet.

Gemäss einer bevorzugten Ausführungsform der vorliegenden Erfindung können wie vorstehend beschrieben eine oder mehrere der vorstehend beschriebenen Membranen oder Träger mit einer Substanz imprägniert sein, welche die Oberflächenspannung von Flüssigkeiten erhöhen kann. Dies führt einerseits zu einem zusätzlichen Filtereffekt und andererseits zu einem besseren Fliessverhalten des Reaktionsgemisches. Es können alle Substanzen eingesetzt werden, die den entsprechenden Effekt auf die Oberflächenspannung ausüben und zur Imprägnierung einer Membran geeignet sind. Erfindungsgemäss bevorzugt werden hierfür polymere Siliciumverbindungen verwendet. Erfindungsgemäss besonders bevorzugt werden Polydimethylsiloxane wie Dimeticon® eingesetzt. Dimeticon® ist eine als Arzneimittel im Handel erhältlicher Substanz gegen Magenbeschwerden. Es hat die chemische Formel

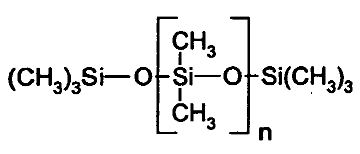

Gemäss einer besonders bevorzugten Ausführungsform der vorliegenden Erfindung umfasst der Schritt der Trennung der Polynukleotide von anderen Probenbestandteilen das in Kontakt bringen der Probe mit einem Träger, vorzugsweise einer Membran, die zur Bindung von Polynukleotiden vorgesehen ist. Die Beschaffenheit dieser Membran ist vorstehend bereits erläutert worden. Erfindungsgemäss bevorzugt werden die unter den erfindungsgemässen Zubereitungsbedingungen negativ geladenen Polynukleotide durch Bereitstellung von funktionellen Gruppen auf der Membran, welche eine positive Ladung besitzen und/oder ausbilden können, an die Membran gebunden. Es können hierfür herkömmliche KationenAustauscherharze als Material für den Filter oder zur Imprägnierung eines Filters aus einem der vorstehend aufgeführten Materialien verwendet werden. Erfindungsgemäss bevorzugt weist diese Membran Aminogruppen wie Diethylaminoethyl-(DEAE)-Gruppen auf.

Die anderen Probenbestandteile, welche nicht an diese Membran binden, treten durch die Membran und werden so von den an die Membran gebundenen Polynukleotiden getrennt. Gemäss einer bevorzugten Ausführungsform der vorliegenden Erfindung werden die nicht an diese Membran gebundenen Probenbestandteile in einen Abfallbehälter geleitet. Unterhalb der Polynukleotide bindenden Membran ist eine Reguliereinheit wie ein Dreiwegehahn bereitgestellt. Dieser ist beim Einfüllen der Probe in die Einheit so eingestellt, dass er durchtretende Substanz in den Abfallbehälter leitet.

Nach Durchtritt der Probe durch die Polynukleotide bindenden Membran und deren Leitung in den Abfallbehälter werden die Polynukleotide gemäss dieser bevorzugten Ausführungsform der vorliegenden Erfindung von der Membran eluiert. Hierzu wird ein Eluiermittel auf die Membran gebracht. Grundsätzlich kann das Eluiermittel durch den Einlass oder eine beliebige andere oberhalb des vorstehenden Trägers vorhandene Öffnung in die Einheit eingeführt werden. Wie vorstehend ausgeführt ist es jedoch erfindungsgemäss bevorzugt, das Eluiermittel in einem Vorratsbehälter einer Einheit zur Zuführung einer Flüssigkeit aufzubewahren und durch Zerstören einer Membran durch Anlegen von Unterdruck in die Einheit einzuführen.

Erfindungsgemäss können herkömmliche Eluiermittel eingesetzt werden. Die Wahl des Eluiermittels ist von der Beschaffenheit der Polynukleotide bindenden Membran abhängig und kann vom Fachmann routinemässig durchgeführt werden. Nicht geeignet für das erfindungsgemässe Verfahren sind jedoch Eluiermittel mit Bestandteilen, welche die nachfolgende chemische Reaktion und Detektion stören. Beispielsweise störend Chelatbildner eine nachfolgende PCR und deren Auswertung. Bei der Zubereitung von Reaktionsgemischen für die PCR sind daher Eluiermittel mit chelatisierenden Bestandteilen zu vermeiden. Erfindungsgemäss bevorzugt wird eine Hochsalzlösung als Eluiermittel eingesetzt. Unter einer Hochsalzlösung wird gemäss der vorliegenden Erfindung eine etwa 0,7-2 M Salzlösung verstanden. Bevorzugt sind jedoch 0,9- 1,8 M Salzlösungen als Eluiermittel. Vorzugsweise weist das erfindungsgemässe Eluiermittel einen pH-Wert von 8,0 bis 8,5 auf, der durch Einstellung mit beispielsweise einem Alkalimetallhydroxid wie Natronlauge erreicht werden kann. Die Menge an Eluiermittel ist abhängig von der im Probenvolumen enthaltenen Menge an Polynukleotiden. Erfindungsgemäss bevorzugt wird im Vorratsbehälter eine Menge von 0,1-1,5 µl Eluiermittel bereitgestellt.

Bei dem erfindungsgemässen Verfahren dieser Ausführungsform wird nach dem Durchtritt der übrigen Probenbestandteile durch die Polynukleotide bindende Membran die Reguliereinheit wie der Dreiwegehahn so verändert, dass nun ankommende Flüssigkeit nicht in den Abfallbehälter, sondern durch den Träger, an welchen die Zusammensetzung gebunden ist, in eine entsprechende Auffangvorrichtung geleitet wird. Gemäss einer bevorzugten Ausführungsform der Einheit mit Vorratsbehälter für das Eluiermittel wird Unterdruck angelegt und somit die Membran der Einheit zur Zuführung des Eluiermittels zerstört. Alternativ wird das Eluiermittel durch den Einlass der Einheit in die Einheit gegeben. Das Eluiermittel gelangt auf die Polynukleotide bindende Membran und eluiert die Polynukleotide von dieser Membran.

Das die Polynukleotide enthaltende Eluat tritt durch den Träger, vorzugsweise die Membran, an welchen die Zusammensetzung gebunden ist. Es kommt zu einer Aufnahme der Zusammensetzung in das Eluat und somit zur Bildung des fertigen Reaktionsgemisches.

Das fertige Reaktionsgemisch tritt durch den Auslass aus der erfindungsgemässen Einheit aus und wird in einer geeigneten Auffangvorrichtung aufgefangen. Es ist hierbei zu beachten, dass die Öffnung zum Anlegen eines Unterdrucks, falls ein solcher angelegt wurde, rechtzeitig verschlossen wird, um einen Durchtritt des Reaktionsgemisches durch diese Öffnung zu vermeiden.

Der gesamte Zubereitungsvorgang des erfindungsgemässen Verfahrens ist somit sehr schnell und wenig arbeitsaufwendig. Typischerweise ist nach 3-5 Minuten das fertige Reaktionsgemisch bereitgestellt. Da der gesamte Vorgang in einer Vorrichtung durchgeführt wird, besteht beim erfindungsgemässen Verfahren nur ein geringes Kontaminierungsrisiko.

Mit der erfindungsgemässen Einheit und dem erfindungsgemässen Verfahren können auch geringe Probenvolumina verarbeitet werden. Das fertige Reaktionsgemisch kann typischerweise ein Volumen von etwa 1 nl bis 2 µl aufweisen. Selbstverständlich können jedoch auch grössere Probenvolumina verarbeitet werden, gegebenenfalls unter Anpassung der vorstehend genannten Mengen an erforderlichem Eluiermittel und Lysemittel.

Die erfindungsgemässe Einheit eignet sich aufgrund der einfachen Bedienung auch für eine automatisierte Zubereitung von Reaktionsgemischen.

Die vorliegende Erfindung betrifft weiterhin eine Vorrichtung zur Zubereitung von Reaktionsmischungen für chemische Reaktionen, insbesondere für die Polymerase-Kettenreaktion, umfassend
a) mindestens eine vorstehend beschriebene Einheit;
b) mindestens eine Reaktionsvorrichtung, welche über eine Öffnung mit dem Auslass einer Einheit in Verbindung steht und nach dem Befüllen mit einem Reaktionsgemisch von der Probenzubereitungsvorrichtung getrennt werden kann.

Gemäss einer bevorzugten Ausführungsform werden mehrere, beispielsweise 3 Einheiten wie beispielsweise Kartuschen in einem Gehäuse bereitgestellt. Bei dem Gehäuse kann es sich beispielsweise um einen Körper aus Kunststoff wie einem Polycarbonatblock handeln. Jede Einheit ist über ihren Auslass mit einer separaten Auffangvorrichtung verbunden. Auf diese Weise können beispielsweise gleichzeitig ein Reaktionsgemisch, eine Positivprobe und eine Negativprobe zubereitet werden.

Die aus den Einheiten austretenden Lösungen gelangen in eine oder mehrere Reaktionsvorrichtungen. Reaktionsvorrichtungen sind gemäss der vorliegenden Erfindung Vorrichtungen, welche zur Durchführung einer chemischen Reaktion wie der PCR und vorteilhaft auch zur Durchführung einer anschliessenden Auswertung geeignet sind. Derartige Reaktionsvorrichtungen sind beispielsweise für die PCR beschrieben. Es wird diesbezüglich auf die US-5,589,136, US-5,639,423 und die US-5,958,349 verwiesen, deren entsprechender Inhalt hiermit ausdrücklich in die vorliegende Anmeldung aufgenommen wird. Eine weitere geeignete Reaktionsvorrichtung ist in der WO 03/019158 beschrieben, deren diesbezüglicher Inhalt hiermit durch Bezugnahme ausdrücklich eingefügt wird.

Gemäss der vorliegenden Erfindung ist die Vorrichtung so ausgestaltet, dass die Einheit von der Reaktionsvorrichtung getrennt werden kann. Eine Ausgestaltung erlaubt die Entnahme der Reaktionsvorrichtung aus der Probenzubereitungsvorrichtung. Die Reaktionsvorrichtung wird über die Einheit befüllt, anschliessend entnommen und in ein Gerät zur Durchführung und gegebenenfalls Auswertung einer chemischen Reaktion wie einer PCR überführt. Eine alternative Ausgestaltung erlaubt die Trennung der Einheit von der restlichen Vorrichtung. In diesem Fall kann sich die Reaktionsvorrichtung in einem Gerät zur Durchführung und gegebenenfalls Auswertung einer chemischen Reaktion wie einer PCR befinden, während sie über die Einheit befüllt wird. Anschliessend wird die Einheit von der Reaktionsvorrichtung getrennt.

Die vorliegende Erfindung wird nachstehend anhand von nicht einschränkenden Zeichnungen und Beispielen veranschaulicht. Es zeigen:
- Fig. 1: eine erste Ausführungsform der erfindungsgemässen Einheit
- Fig. 2: eine zweite Ausführungsform der erfindungsgemässen Einheit
- Fig. 3: eine Ausführungsform der erfindungsgemässen Probenzubereitungsvorrichtung

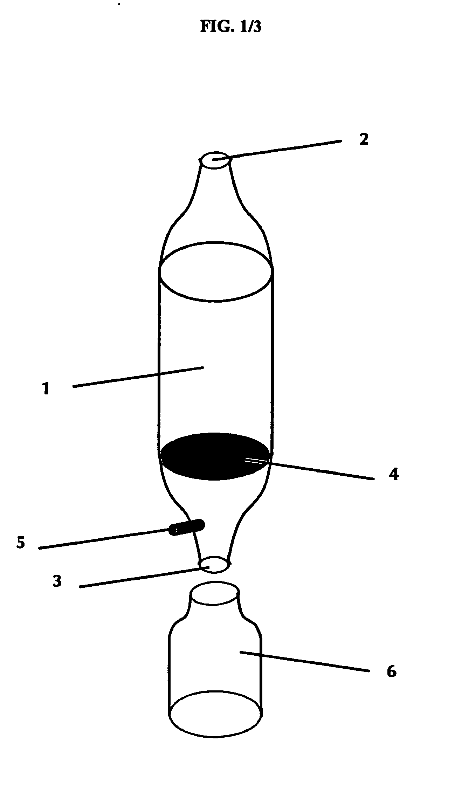

In Fig. 1 ist eine ersten Ausführungsform der erfindungsgemässen Einheit in Form einer Kartusche gezeigt. Es handelt sich hierbei um eine Kartusche zur Zubereitung eines Reaktionsgemisches ohne Lyse- oder Reinigungsschritte. Die Kartusche (1) weist einen Einlass (2) und einen Auslass (3) auf. In der Kartusche (1) ist eine Membran (4) vorhanden, an welche die Zusammensetzung, vorzugsweise das Lyophilisat, gebunden ist. Zusätzlich besitzt die Kartusche (1) eine Öffnung (5) zum Anlegen eines Unterdrucks. Beispielsweise kann an die Öffnung (5) ein Schlauch einer Vakuumpumpe angeschlossen werden. Eine Probe wird durch den Einlass (2) in die Kartusche (1) gegeben. Die Probe tritt durch die Membran (4) und vermengt sich hierbei mit der Zusammensetzung. Anschliessend tritt das fertige Reaktionsgemisch durch den Auslass (3) aus und gelangt in die Auffangvorrichtung (6).

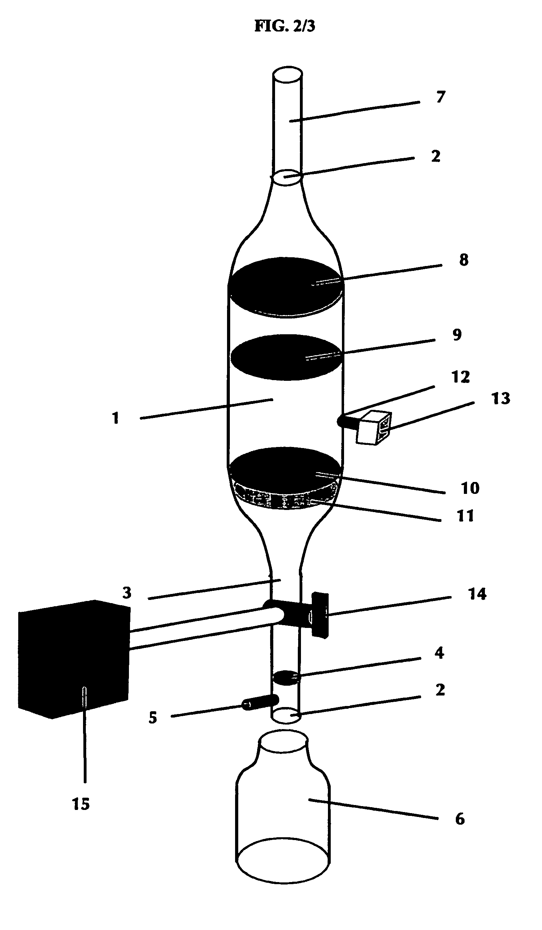

In Fig. 2 ist eine Ausführungsform der erfindungsgemässen Einheit in Form einer Kartusche (1) gezeigt, welche zusätzliche Einrichtungen zur Lyse und Reinigung einer Probe aufweist. Oberhalb des Einlasses (2) der Kartusche (1) ist eine Kapillare (7) optional vorgesehen, um die Einführung definierter Volumina in die Kartusche (1) zu ermöglichen.

Die Probe gelangt zunächst auf einen Filter (8) mit einer Porengrösse, welche den Durchtritt intakter Zellen nicht erlaubt. Es wird nun eine Lyse der Probe durchgeführt. Dies kann entweder durch Zugabe eines Lysemittels durch den Einlass (2) oder durch Bereitstellung eines mit Lysemittels imprägnierten Filters (8) erreicht werden.

Die lysierte Probe tritt anschliessend durch den Filter (9), der mit einem kleineren Porendurchmesser versehen ist als Filter (8). Werden Volumina über 1 ml verarbeitet, so kann optional im Zwischenraum zwischen den Filtern (8) und (9) eine Festkörper absorbierende Substanz bereitgestellt werden. Die Flüssigkeit gelangt so weiter auf den Filter (10). Der Filter (10) ist ein Polynukleotide bindender Filter, der wie vorstehend beschrieben ausgestaltet ist. Die Polynukleotide binden an den Filter (10), während die restlichen Probenbestandteile durch den Filter (10) dringen und durch den Filter (11) in den Abfallbehälter (15) gelangen. Der Dreiwegehahn (14) ist so eingestellt, dass die die Kartusche (1) durchlaufende Substanz in den Abfallbehälter (15) geleitet wird.

Anschliessend wird der Dreiwegehahn (14) so eingestellt, dass nun die Kartusche (1) durchlaufende Substanz in die Auffangvorrichtung (6) geleitet wird. Es wird über die Öffnung (5) ein Unterdruck an die Kartusche (1) angelegt, welcher die Membran (12) der Zuführeinheit zerstört. Aus dem Vorratsbehälter (13) gelangt nun Eluiermittel in die Kartusche (1). Das Eluiermittel eluiert die Polynukleotide von dem Filter (10). Der Filter (10) kann zusätzlich mit einer die Oberflächenspannung erhöhenden Substanz wie einem Polysilan, beispielsweise Dimeticon®, imprägniert sein. Durch diese Imprägnierung wird einerseits ein zusätzlicher Filtereffekt erreicht. Andererseits wird das Imprägniermittel vom Eluat aufgenommen. Das führt dazu, dass das fertige Reaktionsgemisch eine bessere Gängigkeit in Flüssigkeitskanälen aufweist.

Das die Polynukleotide enthaltende Eluat tritt durch den Filter (11) und gelangt auf die Membran (4), an welche die Zusammensetzung gebunden ist. Beim Durchtritt durch die Membran (4) nimmt das Eluat die Zusammensetzung auf, wodurch das fertige Reaktionsgemisch gebildet wird. An die Öffnung (5) sollte nun kein Unterdruck mehr angelegt sein, um ein Durchtreten der Reaktionsgemisches durch die Öffnung (5)zu vermeiden. Das Reaktionsgemisch tritt schliesslich durch den Auslass (3) und gelangt in die Auffangvorrichtung (6).

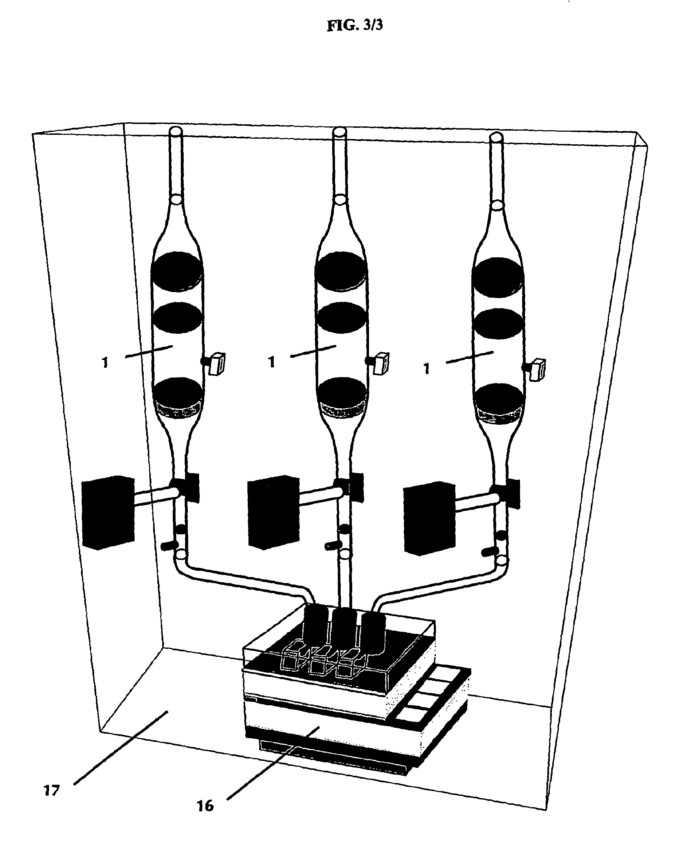

In Fig. 3 ist eine Ausführungsform einer erfindungsgemässen Probenzubereitungsvorrichtung gezeigt. In einem Körper (17), beispielsweise einem Block aus Polycarbonat, sind drei Einheiten wie beispielsweise Kartuschen (1) angeordnet. Diese sind mit einer Reaktionsvorrichtung (16) derart verbunden, dass das aus den jeweiligen Kartuschen (1) austretende Reaktionsgemisch über Leitungen separaten Öffnungen der Reaktionsvorrichtung (16) zugeführt wird. Auf diese Weise lassen sich gleichzeitig mehrere Reaktionsgemische, im vorliegenden Fall drei, zubereiten und in Kammern einer Reaktionsvorrichtung (16) überführen. Anschliessend werden die Kartuschen (1) und der Körper (17) von der Reaktionsvorrichtung (16) getrennt. Im vorliegenden Fall wird die Reaktionsvorrichtung (16) entnommen und in ein Gerät zur Durchführung und Auswertung einer chemischen Reaktion überführt. Wie vorstehend ausgeführt kann gemäss einer anderen Ausführungsform die Reaktionsvorrichtung (16) bereits während des Befüllens im Gerät zur Durchführung und Auswertung einer chemischen Reaktion angeordnet sein. In diesem Fall werden nach dem Zubereiten der Reaktionsmischungen die Kartuschen (1) und der Körper (17) von der Reaktionsvorrichtung (16) abgenommen.

Mit der vorliegenden Erfindung können Reaktionsgemische für chemische Reaktionen auf einfache Weise zubereitet werden. Insbesondere ist das System als Bestandteil der Durchführung von chemischen Reaktionen geeignet, bei denen kontrollierte Temperaturcyclen durchlaufen werden müssen. Ein Beispiel für eine derartige Reaktion ist die einleitend beschriebene Polymerase-Kettenreaktion (PCR).

Mit der vorliegenden Erfindung wird somit die Durchführung von Verfahren erleichtert, mit denen zwischen unterschiedlichen in einer Reaktionsmischung befindlichen Polynukleotiden (DNA oder RNA) differenziert werden soll oder verschiedene Mutationen in Polynukleotiden mit dem erfindungsgemässen System nachgewiesen werden sollen. Somit kann das erfindungsgemässe System zum Nachweis von Protozoen, Pilzen, Bakterien, Viren oder bestimmten DNA-Mutationen eingesetzt werden. Neben der Diagnose von Krankheiten oder Veranlagungen für Krankheiten kann das erfindungsgemässe System somit auch für das Gebiet der Pharmacogenomics verwendet werden, d.h. der individuell nach der genetischen Veranlagung des Patienten ausgelegten Therapie, beziehungsweise für Phytochemie, Veterinärmedizin, Veterinärbiochemie, Mikrobiologie oder allgemein für Gebiete, bei denen Polynukleotidanalytik betrieben werden muss.

### Beispiel 1

Es wurden 500 µl Vollblut in die erfindungsgemässe Kartusche getropft. Das Blut war zusätzlich mit EDTA versetzt, um die Gerinnungskaskade und eine Proteolyse zu verhindern. Das Vollblut wurde zur Entfernung der gröbsten Partikel durch einen Vorfilter (Durchmesser 0.5 µm) gesaugt. Dann wurde die Probe durch einen Nylonfilter (Durchmesser 0.5 µm) als zweite Reinigungsstufe geführt. Das Blut gelangte anschliessend über eine Membran (DEAE-Membran / Hibond), die auf der Unterseite mit einer Dimeticon-Lösung imprägniert war, zum vierten Filter, einem Benetzungsfilter. Nach Passage des Blutes wurde der Hahn von der Stellung "waste" auf die Stellung chip" gedreht. Gleichzeitig wurde das Vakuum erhöht, bis die Membrane mit dem HochsalzPuffer (1M NaCl) platzte. Dieser Puffer gelangte auf den Benetzungsfilter und riss nun die Nukleinsäuren von der Membran weg und führte diese durch einen zweiten Kanal über einen porösen Hartfilter, auf dessen Unterseite das Lyophilisat gebunden war. Das Lyophilisat hatte die vorstehend für die Reaktionslösung beschriebene Zusammensetzung und war aus dieser durch herkömmliche Lyophilisiermethoden erhalten worden. Die Menge an Lyophilisat wurde derart gewählt, dass nach Benetzung eine Endkonzentration an Primer von 0.5 µmol/l, an Anker- und Sensor-Sonden von 0.2-0.4 µmol/l, an Magnesiumchlorid zwischen 1-5 mmol/l, an dNTP, dUTP-UDG entsprechend nach Problemstellung (ca. 2 mmol/l), an Polymerase AmpliTaq Gold (5U) von 7 µl), sowie 8% Trehalose oder Mannose, 1.3% Carbopol 940, 1% Tween 20 erhalten wurden. Nach Durchfluss der Lösung in den Mikrochip gemäss der WO 03/019158 (oder ein anderes Analysesystem) war die Lösung nun bereit für eine PCR-Analytik.

### Beispiel 2

Analog zu Beispiel 1 wurde Vollblut in die erfindungsgemässe Kartusche getropft. Anschliessend wurde die Kartusche verschlossen und während 5 Minuten auf 95°C erhitzt und für 10 Minuten um die eigene Achse zentrifugiert. Anschliessend wurde eine Vakuum angelegt. Das vom Blutkuchen getrennte Plasma konnte ohne Hochsalzlösung direkt über den Benetzungsfilter und den Hartfilter mit dem darauf befindlichen Lyophilisat geführt werden. Nach Durchfluss der Lösung in den Mikrochip gemäss der WO 03/019158 (oder ein anderes Analysesystem) war die Lösung nun bereit für eine PCR-Analytik.

### Beispiel 3

Die Vollblutprobe wurde zunächst für 10 Minuten bei 2500 U/min zentrifugiert. 300 µl des so gewonnenen Blutplasmas wurden einmal mit EDTA versetzt und in die erfindungsgemässe Kartusche getropft, und ein anderes Mal direkt in die erfindungsgemässe Kartusche getropft. Die Probe durchlief die in Beispiel 1 beschriebenen Filter, wobei eine Grobreinigung nicht mehr erforderlich war. Die Probe konnte ohne Hochsalzlösung direkt über den Benetzungsfilter und den Hartfilter mit dem darauf befindlichen Lyophilisat geführt werden. Nach Durchfluss der Lösung in den Mikrochip gemäss der WO 03/019158 (oder ein anderes Analysesystem) war die Lösung nun bereit für eine PCR-Analytik.

### Beispiel 4

Analog zu Beispiel 2 wurde statt einer Vollblutprobe eine Urinprobe verarbeitet und einer PCR-Analytik zugeführt.

### Beispiel 5

Die gemäss Beispiel 1 bis 4 erhaltenen Proben wurden wie in der WO 03/019158 beschrieben einer herkömmlichen PCR-Reaktion unterzogen und ausgewertet. Es wurden sehr zuverlässige und genaue Charakterisierungen von Polynukleotiden erhalten.

## Patentansprüche

1. Lyophilisat zur Durchführung chemischer Reaktionen, insbesondere für die Polymerase-Kettenreaktion, enthaltend
i) eine Polymerase, vorzugsweise DNA-Polymerase, insbesondere Taq-Polymerase;
ii) MgCl₂ sowie gegebenenfalls mindestens ein weiteres Alkali- und/oder Erdalkalimetallhalogenid, vorzugsweise KCl;
iii) Desoxyribonukleotidtriphosphate (dNTPs);
iv) mindestens einen, vorzugsweise zwei Primer;
v) einen Stabilisator, vorzugsweise ein Disaccharid, insbesondere Trehalose;
vi) fluorometrisch erfassbare Stoffe zur Detektion des Reaktionsprodukts;
vii) Carbopol 940; und
viii) gegebenenfalls weitere Zusatzstoffe.

2. Lyophilisat nach Anspruch 1, **dadurch gekennzeichnet, dass** die Stoffe zur Detektion des Reaktionsprodukts Fluoreszenzfarbstoffe sind, die an jeweils ein Anchor- und ein Sensor-Oligonukleotid gebunden sind.

3. Einheit, vorzugsweise Kartusche, zur Zubereitung von Reaktionsgemischen für chemische Reaktionen, insbesondere für die Polymerase-Kettenreaktion, umfassend einen Einlass und einen Auslass sowie mindestens einen Träger, vorzugsweise eine Membran, **dadurch gekennzeichnet, dass**
an mindestens einen Träger das Lyophilisat gemäss Anspruch 1 gebunden ist.

4. Einheit nach Anspruch 3, **dadurch gekennzeichnet, dass** eine Vorrichtung zum Anlegen von Überdruck oder Unterdruck bereitgestellt ist.

5. Einheit nach einem der Ansprüche 3 oder 4, **dadurch gekennzeichnet, dass** oberhalb des Einlasses eine Kapillare angebracht ist.

6. Einheit nach einem der Ansprüche 3 bis 5, **dadurch gekennzeichnet, dass** zwischen dem Einlass und dem Träger, an welchen die Zusammensetzung gemäss einem der Ansprüche 1 oder 2 gebunden ist, eine oder mehrere zusätzliche Membranen oder Träger, vorzugsweise 4 zusätzliche Membranen vorhanden sind.

7. Einheit nach Anspruch 6, **dadurch gekennzeichnet, dass** mindestens ein zusätzlicher Träger, vorzugsweise eine Membran, so ausgebildet ist, vorzugsweise durch Bereitstellung von Diethylaminoethylgruppen, dass Polynukleotide daran gebunden werden können.

8. Einheit nach Anspruch 7, **dadurch gekennzeichnet, dass** die zusätzliche Membran mit einem die Oberflächenspannung einer Flüssigkeit erhöhenden Substanz, vorzugsweise einem Polydimethylsiloxan, imprägniert ist.

9. Einheit nach einem der Ansprüche 6 bis 8, **dadurch gekennzeichnet, dass** in einem Raum zwischen zwei Membranen eine Feststoffe absorbierende Substanz, vorzugsweise Aerosil, bereitgestellt ist.

10. Einheit nach einem der Ansprüche 6 bis 9, **dadurch gekennzeichnet, dass** die dem Einlass am nächsten befindliche zusätzliche Membran mit einem Lysemittel imprägniert ist.

11. Einheit nach einem der Ansprüche 6 bis 10, **dadurch gekennzeichnet, dass** oberhalb den zum Binden von Polynukleotiden vorgesehenen Träger eine Einheit zur Zuführung einer Flüssigkeit, vorzugsweise eine Eluiermittels, bereitgestellt ist.

12. Einheit nach Anspruch 11, **dadurch gekennzeichnet, dass** die Einheit zur Zuführung einer Flüssigkeit durch eine Membran, welche durch Anlegen von Unterdruck durchlässig gemacht werden kann, vom Innenraum der Einheit getrennt ist.

13. Verfahren zur Zubereitung von Reaktionsgemischen für chemische Reaktionen, insbesondere für die Polymerase-Kettenreaktion, umfassend die Schritte:
a) Einführen einer Probe in die Einheit gemäss einem der Ansprüche 3 bis 12;
b) Durchtritt der Probe durch einen Träger, vorzugsweise eine Membran, an welche ein Lyophilisat gemäss einem der Ansprüche 1 oder 2 gebunden ist, so dass das fertige Reaktionsgemisch aus dem Auslass der Einheit austritt.

14. Verfahren nach Anspruch 13, **dadurch gekennzeichnet, dass** zwischen den Schritten a) und b) als zusätzliche Schritte
a1) eine Lyse von Zellen in der Probe
a2) die Trennung der Polynukleotide von anderen Probenbestandteilen durchgeführt werden.

15. Verfahren nach Anspruch 14, **dadurch gekennzeichnet, dass** der Schritt a1) durch in Kontakt bringen der Probe mit einer mit Lysemittel imprägnierten Membran erfolgt.

16. Verfahren nach einem der Ansprüche 14 oder 15, **dadurch gekennzeichnet, dass** der Schritt a2) das Binden der Polynukleotide an eine Membran, die Abtrennung der übrigen Probenbestandteile und das anschliessende Eluieren der Polynukleotide von dieser Membran mit Hilfe eines Eluiermittels, vorzugsweise einer Hochsalzlösung, welche keine chelatbildenden Substanzen enthält, umfasst.

17. Verfahren nach einem der Ansprüche 14 bis 16, **dadurch gekennzeichnet, dass** beim Schritt a2) die Probe durch 1 oder mehrere Membranen, vorzugsweise 4 Membranen geleitet wird.

18. Verfahren nach einem der Ansprüche 14 bis 17, **dadurch gekennzeichnet, dass** beim Schritt a2) die Probe durch eine Feststoffe absorbierende Substanz geleitet wird.

19. Vorrichtung zur Zubereitung von Reaktionsmischungen für chemische Reaktionen, insbesondere für die Polymerase-Kettenreaktion, umfassend
a) mindestens eine, vorzugsweise drei Einheiten, vorzugsweise Kartuschen, gemäss einem der Ansprüche 3 bis 12;
b) mindestens eine Reaktionsvorrichtung, welche über eine Öffnung mit dem Auslass einer Einheit in Verbindung steht und nach dem Befüllen mit einem Reaktionsgemisch von der Probenzubereitungsvorrchtung getrennt werden kann.

20. Vorrichtung nach Anspruch 19, **dadurch gekennzeichnet, dass** die mindestens eine Reaktionsvorrichtung nach dem Befüllen mit einem Reaktionsgemisch aus der Probenzubereitungsvorrichtung entnommen und in eine Vorrichtung zur Durchführung und gegebenenfalls Auswertung einer chemischen Reaktion, vorzugsweise einer Polymerase-Kettenreaktion, überführt werden kann.

21. Vorrichtung nach Anspruch 19 oder 20, weiterhin umfassend eine Vorrichtung zur Durchführung und Auswertung einer chemischen Reaktion, vorzugsweise einer Polymerase-Kettenreaktion, **dadurch gekennzeichnet, dass** die mindestens eine Einheit nach dem Befüllen der Reaktionsvorrichtung von der restlichen Vorrichtung getrennt werden kann.

22. Verwendung eines Lyophilisats gemäss einem der Ansprüche 1 oder 2 zur Zubereitung von Reaktionsmischungen für chemische Reaktionen, insbesondere für die Polymerase-Kettenreaktion.

23. Verwendung einer Einheit gemäss einem der Ansprüche 3 bis 12 zur Zubereitung von Reaktionsmischungen für chemische Reaktionen, insbesondere für die Polymerase-Kettenreaktion.

24. Verwendung einer Probenzubereitungsvorrichtung gemäss einem der Ansprüche 19 bis 21 zur Zubereitung von Reaktionsmischungen für chemische Reaktionen, insbesondere für die Polymerase-Kettenreaktion.

25. Verfahren zur Identifizierung von Polynukleotiden, vorzugsweise DNA, in einer Probe, umfassend die Zubereitung eines Reaktionsgemisches für eine Polymerase-Kettenreaktion in einer Einheit gemäss einem der Ansprüche 3 bis 12, Überführung des Reaktionsgemisches in eine Reaktionsvorrichtung und Durchführung und Auswertung einer Polymerase-Kettenreaktion.

## Claims

1. Lyophilizate for carrying out chemical reactions, in particular for the polymerase chain reaction, comprising
i) a polymerase, preferably DNA polymerase, especially Taq polymerase;
ii) MgCl₂ and, optionally, at least one further alkali metal and/or alkaline earth metal halide, preferably KCl;
iii) deoxyribonucleotide triphosphates (dNTPs);
iv) at least one, preferably two primers;
v) a stabilizer, preferably a disaccharide, especially trehalose;
vi) substances detectable by fluorometry for detection of the reaction product;
vii) Carbopol 940; and
viii)optionally further additives.

2. Lyophilizate according to Claim 1, **characterized in that** the substances for detection of the reaction product are fluorescent dyes which are bound to in each case an anchor oligonucleotide and a sensor oligonucleotide.

3. Unit, preferably cartridge, for preparing reaction mixtures for chemical reactions, especially for the polymerase chain reaction, comprising an inlet and an outlet, and at least one support, preferably a membrane, **characterized in that** the lyophilizate according to Claim 1 is bound to at least one support.

4. Unit according to Claim 3, **characterized in that** a device for applying elevated pressure or reduced pressure is provided.

5. Unit according to either of Claims 3 or 4, **characterized in that** a capillary is attached above the inlet.

6. Unit according to any of Claims 3 to 5, **characterized in that** one or more additional membranes or supports, preferably 4 additional membranes, are present between the inlet and the support to which the composition according to either of Claims 1 or 2 is bound.

7. Unit according to Claim 6, **characterized in that** at least one additional support, preferably a membrane, is designed, preferably by providing diethylaminoethyl groups, so that polynucleotides can be bound thereto.

8. Unit according to Claim 7, **characterized in that** the additional membrane is impregnated with a substance which increases the surface tension of a liquid, preferably with a polydimethylsiloxane.

9. Unit according to any of Claims 6 to 8, **characterized in that** a substance which absorbs solids, preferably Aerosil, is provided in the space between two membranes.

10. Unit according to any of Claims 6 to 9, **characterized in that** the additional membrane located closest to the inlet is impregnated with a lysing agent.

11. Unit according to any of Claims 6 to 10, **characterized in that** a unit for supplying a liquid, preferably an eluent, is provided above the support provided for binding polynucleotides.

12. Unit according to Claim 11, **characterized in that** the unit for supplying a liquid is separated from the interior of the unit by a membrane, which can be made permeable on application of reduced pressure.

13. Method for preparing reaction mixtures for chemical reactions, in particular for the polymerase chain reaction, comprising the steps:
a) introducing a sample into the unit according to any of Claims 3 to 12;
b) passing the sample through a support, preferably a membrane, to which a lyophilizate according to either of Claims 1 or 2 is bound, so that the finished reaction mixture emerges from the outlet of the unit.

14. Method according to Claim 13, **characterized in that** between steps a) and b) the additional steps of
a1) lysis of cells in the sample
a2) separation of the polynucleotides from other sample constituents
are carried out.

15. Method according to Claim 14, **characterized in that** step a1) takes place by contacting the sample with a membrane impregnated with lysing agent.

16. Method according to either of Claims 14 or 15, **characterized in that** step a2) comprises binding the polynucleotides to a membrane, removal of the other sample constituents and subsequent elution of the polynucleotides from this membrane with the aid of an eluent, preferably a high-salt solution which comprises no chelating substances.

17. Method according to any of Claims 14 to 16, **characterized in that** in step a2) the sample is guided through 1 or more membranes, preferably 4 membranes.

18. Method according to any of Claims 14 to 17, **characterized in that** in step a2) the sample is guided through a substance which absorbs solids.

19. Device for preparing reaction mixtures for chemical reactions, in particular for the polymerase chain reaction, comprising
a) at least one, preferably three units, preferably cartridges, according to any of Claims 3 to 12;
b) at least one reaction device which is connected via an aperture to the outlet of a unit and, after charging with a reaction mixture, can be separated from the sample preparation device.

20. Device according to Claim 19, **characterized in that** at least one reaction device can, after the charging with a reaction mixture, be taken out of the sample preparation device and transferred into a device for carrying out and optionally, evaluating a chemical reaction, preferably a polymerase chain reaction.

21. Device according to Claim 19 or 20, further comprising a device for carrying out and evaluating a chemical reaction, preferably a polymerase chain reaction, **characterized in that** the at least one unit can, after charging of the reaction device, be separated from the remaining device.

22. Use of a lyophilizate according to either of Claims 1 or 2 for preparing reaction mixtures for chemical reactions, in particular for the polymerase chain reaction.

23. Use of a unit according to any of Claims 3 to 12 for preparing reaction mixtures for chemical reactions, in particular for the polymerase chain reaction.

24. Use of a sample preparation device according to any of Claims 19 to 21 for preparing reaction mixtures for chemical reactions, in particular for the polymerase chain reaction.

25. Method for identifying polynucleotides, preferably DNA, in a sample, comprising the preparation of a reaction mixture for a polymerase chain reaction in a unit according to any of Claims 3 to 12, transferring the reaction mixture into a reaction device and carrying out and evaluating a polymerase chain reaction.

## Revendications

1. Lyophilisat pour la réalisation de réactions chimiques, en particulier pour la réaction en chaîne par polymérase, contenant
i) une polymérase, de préférence une ADN-polymérase, en particulier la Taq-polymérase ;
ii) du MgCl₂ ainsi que le cas échéant au moins un autre halogénure de métal alcalin et/ou de métal alcalino-terreux, de préférence KCl ;
iii) des désoxyribonucléotide-triphosphates (dNTPs) ;
iv) au moins un, de préférence deux amorces ;
v) un stabilisateur, de préférence un disaccharide, en particulier le tréhalose ;
vi) des substances détectables par fluorimétrie pour la détection du produit de réaction ;
vii) du Carbopol 940 ; et
viii) le cas échéant d'autres additifs.

2. Lyophilisat selon la revendication 1, **caractérisé en ce que** les substances pour la détection du produit de réaction sont des colorants de fluorescence, qui sont à chaque fois liés à un oligonucléotide d'ancrage et un oligonucléotide capteur.

3. Unité, de préférence cartouche, destinée à la préparation de mélanges réactionnels pour des réactions chimiques, en particulier pour la réaction en chaîne par polymérase, comprenant une admission et une sortie ainsi qu'au moins un support, de préférence une membrane, **caractérisée en ce que** le lyophilisat selon la revendication 1 est lié sur au moins un support.

4. Unité selon la revendication 3, **caractérisée en ce qu'**un dispositif pour appliquer une surpression ou une dépression est mis à disposition.

5. Unité selon l'une quelconque des revendications 3 ou 4, **caractérisée en ce qu'**un capillaire est agencé au-dessus de l'admission.

6. Unité selon l'une quelconque des revendications 3 à 5, **caractérisée en ce qu'**un(e) ou plusieurs membranes ou supports supplémentaires, de préférence 4 membranes supplémentaires, sont présent(e)s entre l'admission et le support auquel la composition selon l'une quelconque des revendications 1 ou 2 est liée.

7. Unité selon la revendication 6, **caractérisée en ce qu'**au moins un support supplémentaire, de préférence une membrane, est réalisé de manière telle que des polynucléotides peuvent y être liés, de préférence par mise à disposition de groupes diéthylaminoéthyle.

8. Unité selon la revendication 7, **caractérisée en ce que** la membrane supplémentaire est imprégnée d'une substance augmentant la tension superficielle d'un liquide, de préférence un polydiméthylsiloxane.

9. Unité selon l'une quelconque des revendications 6 à 8, **caractérisée en ce qu'**une substance absorbant les solides, de préférence l'Aerosil, est mise à disposition dans un espace entre deux membranes.

10. Unité selon l'une quelconque des revendications 6 à 9, **caractérisée en ce que** la membrane supplémentaire la plus proche de l'admission est imprégnée d'un agent de lyse.

11. Unité selon l'une quelconque des revendications 6 à 10, **caractérisée en ce qu'**une unité destinée à l'alimentation d'un liquide, de préférence un éluant, est mise à disposition au-dessus du support prévu pour la liaison de polynucléotides.

12. Unité selon la revendication 11, **caractérisée en ce que** l'unité pour l'alimentation d'un liquide est séparée de l'espace intérieur de l'unité par une membrane qui peut être rendue perméable par l'application d'une dépression.

13. Procédé pour la préparation de mélanges réactionnels pour des réactions chimiques, en particulier pour la réaction en chaîne par polymérase, comprenant les étapes :
a) introduction d'un échantillon dans l'unité selon l'une quelconque des revendications 3 à 12 ;
b) passage de l'échantillon à travers un support, de préférence une membrane, auquel est lié un lyophilisat selon l'une quelconque des revendications 1 ou 2 de manière telle que le mélange réactionnel fini sort de la sortie de l'unité.

14. Procédé selon la revendication 13, **caractérisé en ce qu'**on réalise entre les étapes a) et b), comme étapes supplémentaires
a1) une lyse des cellules dans l'échantillon
a2) la séparation des polynucléotides d'autres constituants de l'échantillon.

15. Procédé selon la revendication 14, **caractérisé en ce que** l'étape a1) a lieu par mise en contact de l'échantillon avec une membrane imprégnée de l'agent de lyse.

16. Procédé selon l'une quelconque des revendications 14 ou 15, **caractérisé en ce que** l'étape a2) comprend la liaison des polynucléotides à une membrane, la séparation des autres constituants de l'échantillon et l'élution consécutive des polynucléotides de cette membrane à l'aide d'un éluant, de préférence une solution hautement saline, qui ne contient pas de substances chélatantes.

17. Procédé selon l'une quelconque des revendications 14 à 16, **caractérisé en ce que** lors de l'étape a2) l'échantillon est guidé à travers 1 ou plusieurs membranes, de préférence 4 membranes.

18. Procédé selon l'une quelconque des revendications 14 à 17, **caractérisé en ce que** lors de l'étape a2), l'échantillon est guidé à travers la substance absorbant des solides.

19. Dispositif pour la préparation de mélanges réactionnels pour des réactions chimiques, en particulier pour la réaction en chaîne par polymérase, comprenant
a) au moins une, de préférence trois unités, de préférence des cartouches, selon l'une quelconque des revendications 3 à 12 ;
b) au moins un dispositif de réaction, qui est assemblé, via une ouverture, à la sortie d'une unité et qui peut être séparé après le remplissage par un mélange réactionnel du dispositif de préparation d'échantillons.

20. Dispositif selon la revendication 19, **caractérisé en ce que** ledit au moins un dispositif de réaction peut être prélevé après le remplissage par un mélange réactionnel du dispositif de préparation d'échantillons et transféré dans un dispositif pour la réalisation et le cas échéant l'évaluation d'une réaction chimique, de préférence une réaction en chaîne par polymérase.

21. Dispositif selon la revendication 19 ou 20, comprenant en outre un dispositif pour la réalisation et l'évaluation d'une réaction chimique, de préférence une réaction en chaîne par polymérase, **caractérisé en ce que** ladite au moins une unité peut être séparée du reste du dispositif après le remplissage du dispositif de réaction.

22. Utilisation d'un lyophilisat selon l'une quelconque des revendications 1 ou 2 pour la préparation de mélanges réactionnels pour des réactions chimiques, en particulier pour la réaction en chaîne par polymérase.

23. Utilisation d'une unité selon l'une quelconque des revendications 3 à 12 pour la préparation de mélanges réactionnels pour des réactions chimiques, en particulier pour la réaction en chaîne par polymérase.

24. Utilisation d'un dispositif de préparation d'échantillons selon l'une quelconque des revendications 19 à 21 pour la préparation de mélanges réactionnels pour des réactions chimiques, en particulier pour la réaction en chaîne par polymérase.

25. Procédé pour l'identification de polynucléotides, de préférence l'ADN, dans un échantillon comprenant la préparation d'un mélange réactionnel pour une réaction en chaîne par polymérase dans une unité selon l'une quelconque des revendications 3 à 12, le transfert du mélange réactionnel dans un dispositif de réaction et la réalisation et l'évaluation d'une réaction en chaîne par polymérase.
